(19) Europäisches Patentamt
European Patent Office
Office européen des brevets

(11) Publication number: **0 098 713**
**B1**

# (12) EUROPEAN PATENT SPECIFICATION

(45) Date of publication of patent specification: 12.08.87

(21) Application number: 83303583.5

(22) Date of filing: 22.06.83

(51) Int. Cl.⁴: C 07 D 295/18, C 07 D 215/48, C 07 D 217/26, C 07 D 237/26, C 07 D 241/44, C 07 D 249/08, C 07 D 261/10, C 07 D 307/80, C 07 D 307/84, C 07 D 311/04, A 61 K 31/495

## (54) Benzoylpiperazine esters and a process for their production.

(30) Priority: 25.06.82 JP 109192/82
28.04.83 JP 75868/83

(43) Date of publication of application:
18.01.84 Bulletin 84/03

(45) Publication of the grant of the patent:
12.08.87 Bulletin 87/33

(84) Designated Contracting States:
AT BE CH DE FR GB IT LI NL SE

(56) References cited:
EP-A-0 071 433

CHEMICAL ABSTRACTS, vol. 96, no. 21, 24th May 1982, page 681, no. 180955d, Columbus, Ohio, US

**The file contains technical information submitted after the application was filed and not included in this specification**

(73) Proprietor: KOWA COMPANY, LTD.
6-29, 3-chome Nishiki
Naka-ku Nagoya Aichi-ken (JP)

(72) Inventor: Fujii, Setsuo
1-4-27, Midorigaoka
Toyonaka-shi Osaka-fu (JP)
Inventor: Hattori, Eizuo
2-6-21, Tsurumai
Sakado-shi Saitama-ken (JP)
Inventor: Hirata, Mitsuteru
T.R.L. Kowa Co. 2-17-43 Noguchi-cho
Higashi-Murayama-shi Tokyo (JP)
Inventor: Watanabe, Koichiro
T.R.L. Kowa Co. 2-17-43 Noguchi-cho
Higashi-Murayama-shi Tokyo (JP)
Inventor: Onogi, Kazuhiro
T.R.L. Kowa Co. 2-17-43 Noguchi-cho
Higashi-Murayama-shi Tokyo (JP)
Inventor: Nagakura, Masahiko
T.R.L. Kowa Co. 2-17-43 Noguchi-cho
Higashi-Murayama-shi Tokyo (JP)

(74) Representative: Webb, Frederick Ronald et al
G.F. Redfern & Co. Marlborough Lodge 14
Farncombe Road
Worthing West Sussex BN11 2BT (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 098 713 B1

Courier Press, Leamington Spa, England.

**Description**

This invention relates to novel benzoylpiperazine esters and to a process for producing such esters.

The inventors of the present invention have previously found that various phenyl esters have excellent chymotrypsin inhibitive effects (see Japanese Patent Specification No. 158737/1981 as laid open, and EP—A—71433).

The inventors have further synthesized analogous compounds to study their pharmacological effects. In the studies leading to the present invention, it has been found that certain benzoylpiperazine esters and acid addition salts thereof exert even better chymotrypsin inhibitive effects.

Accordingly, it is an object of the present invention to provide benzoylpiperazine esters and acid addition salts thereof which possess substantially superior chymotrypsin inhibitive activity and can be widely used, for example, as medicines such as those for the therapy of pancreatopathy.

Another object of the invention is to provide a process for producing such esters.

According to one aspect of the invention, there are provided benzoylpiperazine esters and acid addition salts thereof, said esters being represented by the general formula (I):—

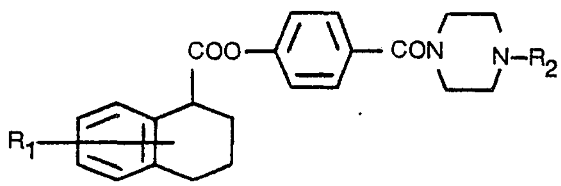

(I)

where $R_1$ is hydrogen or a methoxy group, and $R_2$ is a straight or branched-chain $C_1$—$C_6$ alkyl, cyclohexyl, cyclohexylmethyl, or benzyl group, but excluding the compound where $R_1$ is H and $R_2$ is $CH_3$.

The compounds of the formula (I) can be produced by esterification of carboxylic acids of the formula (II) below with substituted phenols of the formula (III) below, in accordance with the following reaction scheme,

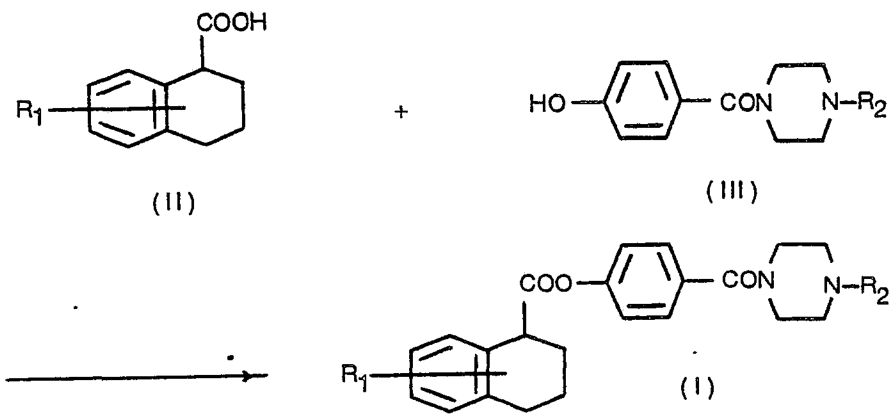

(II) + (III) → (I)

wherein the symbols A, $R_1$ and $R_2$ each have the same meaning as before.

The esterification reaction between the compounds of the formulae (II) and (III) may be carried out using any conventional technique. Suitable techniques useful in the invention include reacting the compound (II), or a reactive derivative thereof, for example, an acid halide, an acid anhydride, a mixed acid anhydride, an active ester, or an active azide, with the compound (III), or a reactive derivative thereof; and reacting the compounds (II) and (III) in the presence of a dehydrating agent, such as dicyclohexyl carbodiimide.

The compound (I) obtained in this way may be further converted by a conventional method to an inorganic acid salt, for example, of hydrochloric acid, sulfuric acid, phosphoric acid, or hydrobromic acid; or an organic acid salt, for example, of acetic acid, propionic acid, maleic acid, fumaric acid, tartaric acid, citric acid, methane sulfonic acid, benzene sulfonic acid, or toluene sulfonic acid.

The chymotrypsin inhibitive activity of the compounds according to the invention will be readily understood by reference to the following test results.

Measuring Method

A solution prepared by mixing 0.1 ml of dimethylsulfoxide solution containing a compound to be tested, 0.1 ml of water and 0.1 ml of a buffer solution containing 10 µg/ml of chymotrypsin (0.1M tris-hydrochloric acid buffer solution, pH 8.0) was incubated for 10 minutes by the method of Muramatsu et. al.

[see the Journal of Biochemistry, 62, 408 (1967)]. 0.2 ml of a buffer solution containing 25 mM of an acetyl-L-tryosine ethyl ester was mixed with the above-prepared solution and reacted at 30°C for 30 minutes. The amount of the remaining substrate was determined by causing the same to develop color by the Hestrin Method and measuring the absorbance at 530 nm.

For comparative purposes, use was made of tosylphenylalanine chloromethyl ketone which was known as a chymotrypsin inhibitor (Comparative Compound I).

The results are as shown in Table 1.

TABLE 1

| Test compounds | Chymotrypsin inhibitive activity (50% inhibition concentration (M) |
|---|---|
| 1 | $8 \times 10^{-7}$ |
| 2 | $9 \times 10^{-7}$ |
| 3 | $8 \times 10^{-7}$ |
| 4 | $9 \times 10^{-7}$ |
| Comparative compound I | $5 \times 10^{-4}$ |

Note: The number for each test compound of the invention indicates the corresponding example as will appear hereinafter.

The invention is illustrated by the following specific examples which are provided for purposes of illustration only and are not to be regarded as limiting.

Example 1

1-Methyl-4-(4-(7-methoxy-1,2,3,4-tetrahydro-1-naphthoyloxy)benzoyl)piperazine.methanesulfonate:

To a 100 ml solution of acetonitrile containing 4.4 g of 1-methyl-4-(4-hydroxybenzoyl)piperazine and 4.94 g of 7-methoxy-1,2,3,4-tetrahydro-1-naphthylcarboxylic acid was added 4.94 g of dicyclohexyl carbodiimide, and the mixture was stirred overnight at room temperature. After removal of any insoluble matter by filtration, the filtrate was concentrated under reduced pressure, incorporated with 50 ml of 0.5N hydrochloric acid and washed with ethyl acetate. After being neutralised with a saturated solution of sodium bicarbonate, the aqueous phase was extracted with ethyl acetate. The extract was washed with water and dried, followed by removal of the solvent by distillation, to obtain a crude oily product. Purification of the product by silica gel column chromatography (eluting solution:chloroform — methanol 30:1) gave 7.57 g of an oily product. The oily product was further converted in a conventional manner to methanesulfonate, thereby obtaining 6.2 g of needle-like pale yellow crystals having a melting point of 150—151°C (yield: 61.6%).

Examples 2—9

The same procedure as in Example 1 was repreated to obtain several compounds as shown in Table 2.

TABLE 2

| Example | In Formula (I) 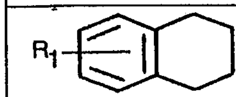 | R₂ | Acid Addition Salt | Yield (%) | Appearance | Melting Point (°C) |
|---|---|---|---|---|---|---|
| 2 | 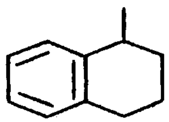 | 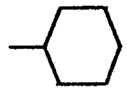 | $CH_3SO_3H$ | 86.2 | Needle-like colorless crystals | 205—207 |
| 3 | 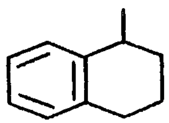 | 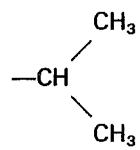 | $CH_3SO_3H$ | 89.7 | Needle-like colorless crystals | 180—182 |
| 4 | 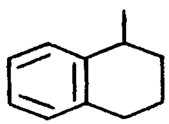 | $—CH_2CH_3$ | $CH_3SO_3H$ | 69.2 | Needle-like colorless crystals | 140—143 |
| 5 | 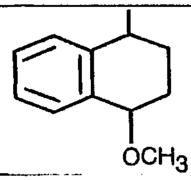 | $—CH_3$ | — | 56.8 | Colorless oils | — |
| 6 | 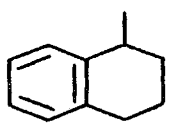 | 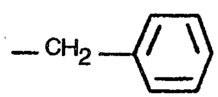 | $CH_3SO_3H$ | 77.4 | Needle-like colorless crystals | 180—190 |
| 7 | 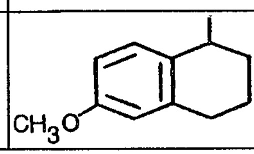 | $—CH_3$ | — | 42.2 | Oils | — |

TABLE 2 continued

| Example | In Formula (I) | | Acid Addition Salt | Yield (%) | Appearance | Melting Point (°C) |
|---|---|---|---|---|---|---|
| | 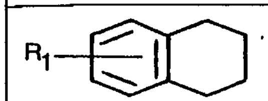 | $R_2$ | | | | |
| 8 | 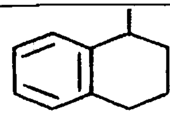 | $-CH_2(CH_2)_4CH_3$ | $CH_3SO_3H$ | 52.5 | Pale yellow crystals | 153—155 |
| 9 | 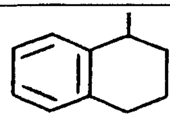 | $-CH_2-$ 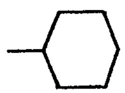 | $CH_3SO_3H$ | 55.4 | Colorless crystals | 227—229 |

**Claims for the Contracting States: GB DE FR CH IT NL BE SE LI**

1. Benzoylpiperazine esters and acid salts thereof, said esters being represented by the general formula:—

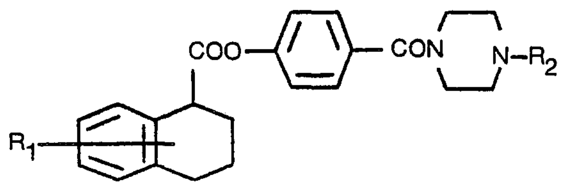

where $R_1$ is hydrogen or a methoxy group, and $R_2$ is a straight or branched-chain $C_1$—$C_6$ alkyl, cyclohexyl, cyclohexylmethyl, or benzyl group, but excluding the compound where $R_1$ is H and $R_2$ is $CH_3$.

2. A process for producing a benzoyl-piperazine ester represented by the general formula (I):

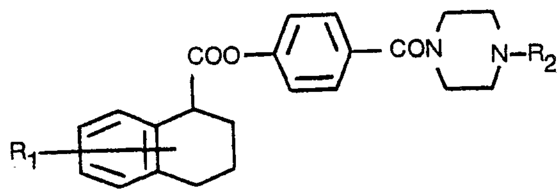

(I)

where $R_1$ is hydrogen or a methoxy group and $R_2$ is a straight- or branched-chain $C_1$—$C_6$ alkyl group, or a cyclohexyl, cyclohexylmethyl or benzyl group, but excluding the compound where $R_1$ is H and $R_2$ is $CH_3$, which process comprises reacting a carboxylic acid represented by the general formula:

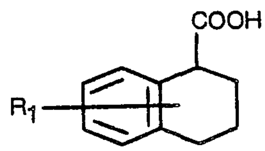

wherein $R_1$ has the same meaning as above, or a reactive derivative thereof, with a substituted phenol represented by the general formula:

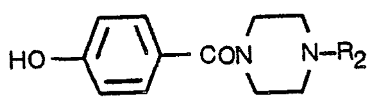

where $R_2$ has the same meaning as above, or a reactive derivative thereof.

**Claims for the Contracting State: AT**

A process for producing a benzoyl-piperazine ester represented by the general formula (I):

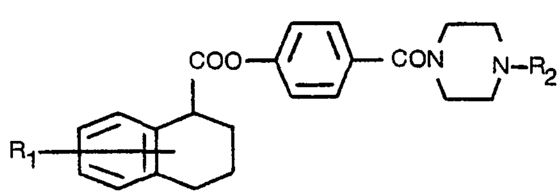

(I)

where $R_1$ is hydrogen or a methoxy group and $R_2$ is a straight- or branched-chain $C_1$—$C_6$ alkyl group, or a cyclohexyl, cyclohexylmethyl or benzyl group, but excluding the compound where $R_1$ is H and $R_2$ is $CH_3$, which process comprises reacting a carboxylic acid represented by the general formula:

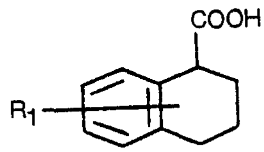

wherein $R_1$ has the same meaning as above, or a reactive derivative thereof, with a substituted phenol represented by the general formula:

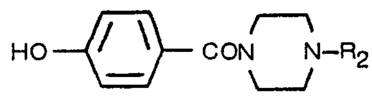

where $R_2$ has the same meaning as above, or a reactive derivative thereof.

**Patentansprüche für die Vertragsstaaten: GB DE FR CH IT NL BE SE LI**

1. Benzoylpiperazinester und deren Salze, wobei die Ester durch die allgemeine Formel

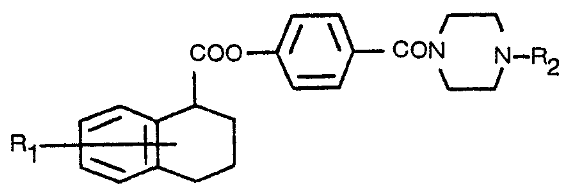

repräsentiert, werden, wobei $R_1$ Wasserstoff oder eine Methoxygruppe und $R_2$ eine gerade oder verzweigte $C_1$—$C_6$ Alkyl-, Cyclohexyl-, Cyclohexylmethyl- oder Benzylgruppe ist, wobei jedoch die Verbindung, bei der $R_1$ H und $R_2$ $CH_3$ ist, ausgeschlossen ist.

2. Verfahren zur Herstellung eines Benzoylpiperazinesters, entsprechend der allgemeinen Formel (I):

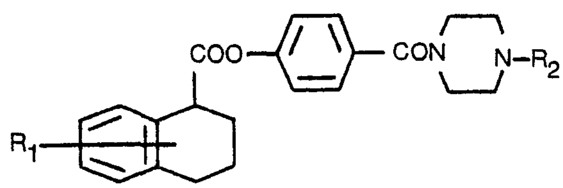

(I)

wobei $R_1$ ein Wasserstoff oder eine Methoxygruppe und $R_2$ eine gerade oder verzweigte $C_1$—$C_6$ Alkyl-, Cyclohexyl-, Cyclohexylmethyl- oder Benzylgruppe ist, wobei jedoch die Verbindung, bei der $R_1$ H und $R_2$ $CH_3$ ist, ausgenommen ist, wobei das Verfahren die Reaktion einer Carboxylsäure, repräsentiert durch die allgemeine Formel:

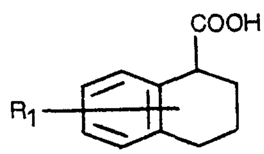

worin $R_1$ die oben definierte Bedeutung hat, oder eines reaktiven Derivats mit einem substituierten Phenol, entsprechend der allgemeinen Formel:

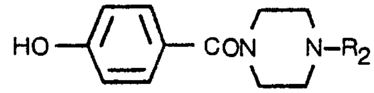

worin $R_2$ die oben angegebene Bedeutung hat, oder einem reaktiven Derivat umfaßt.

**Patentanspruch für den Vertragsstaat AT**

Verfahren zur Herstellung eines Benzoylpiperazinesters, entsprechend der allgemeinen Formel (I):

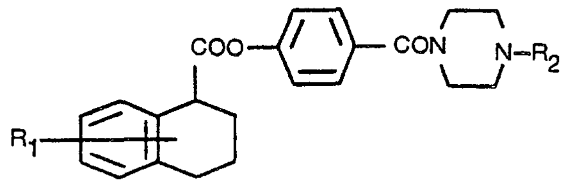

(I)

wobei $R_1$ ein Wasserstoff oder eine Methoxygruppe und $R_2$ eine gerade oder verzweigte $C_1$—$C_6$ Alkyl-, Cyclohexyl-, Cyclohexylmethyl- oder Benzylgruppe ist, wobei jedoch die Verbindung, bei der $R_1$ H und $R_2$ $CH_3$ ist, ausgenommen ist, wobei das Verfahren die Reaktion einer Carboxylsäure, repräsentiert durch die allgemeine Formel:

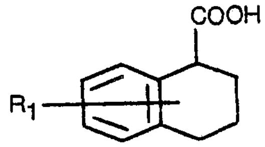

worin $R_1$ die oben definierte Bedeutung hat, oder eines reaktives Derivats mit einem substituierten Phenol, entsprechend der allgemeinen Formel:

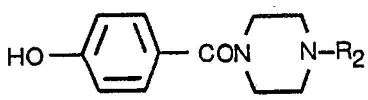

worin $R_2$ die oben angegebene Bedeutung hat, oder einem reaktiven Derivat umfaßt.

**Revendications pour les Etats contractants: BE CH DE FR GB IT LI NL SE**

1. Esters de benzoylpipérazine et leurs sels acides, ces esters étant représentés par la formule générale:

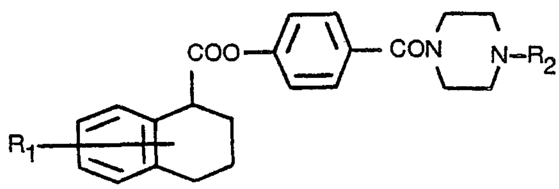

dans laquelle $R_1$ représente un atome d'hydrogène ou un groupe méthoxy et $R_2$ représente un groupe alkyle en $C_1$—$C_6$ à chaîne droite ou ramifiée, un groupe cyclohexyle, cyclohexylméthyle ou benzyle, mais à l'exclusion du composé dans lequel $R_1$ représente H et $R_2$ représente $CH_3$.

2. Procédé de préparation d'un ester de benzoylpipérazine représenté par la formule générale (1):

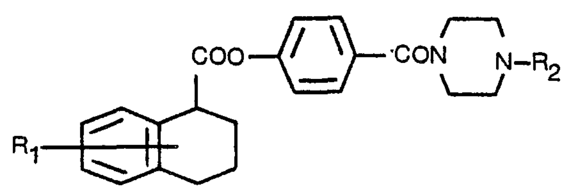

(I)

dans laquelle $R_1$ représente un atome d'hydrogène ou un groupe méthoxy et $R_2$ représente un groupe alkyle en $C_1$—$C_6$ à chaîne droite ou ramifiée, un groupe cyclohexyle, cyclohexylméthyle ou benzyle, mais à l'exclusion du composé dans lequel $R_1$ représente H et $R_2$ représente $CH_3$, ledit procédé consistant à faire réagir un acide carboxylique représenté par la formule générale:

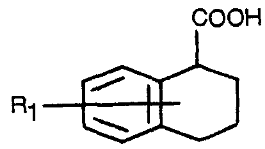

dans laquelle $R_1$ est défini comme ci-dessus, ou un de ces dérivés réactifs, avec un phénol substitué représenté par la formule générale:

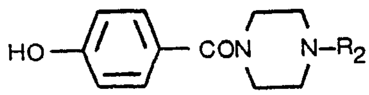

dans laquelle $R_2$ est défini comme ci-dessus, ou avec un de ses dérivés réactifs.

**Revendications pour l'Etat contractant: AT**

Procédé de préparation d'un ester de benzoylpipérazine représenté par la formule générale (I):

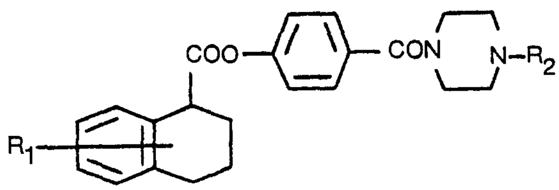

dans laquelle $R_1$ représente un atome d'hydrogène ou un groupe méthoxy et $R_2$ représente un groupe alkyle en $C_1$—$C_6$ à chaîne droite ou ramifiée, un groupe cyclohexyle, cyclohexylméthyle ou benzyle, mais à l'exclusion du composé dans lequel $R_1$ représente H et $R_2$ représente $CH_3$, ledit procédé consistant à faire réagir un acide carboxylique représenté par la formule générale:

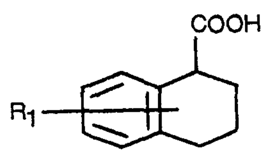

dans laquelle $R_1$ est défini comme ci-dessus, ou un de ces dérivés réactifs, avec un phénol substitué représenté par la formule générale:

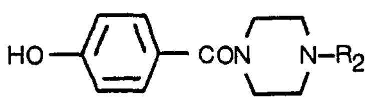

dans laquelle $R_2$ est défini comme ci-dessus, ou avec un de ses dérivés réactifs.